# NEUE EUROPÄISCHE PATENTSCHRIFT

(11) **EP 0 906 257 B2**
(45) Veröffentlichungstag und Bekanntmachung des Hinweises auf die Entscheidung über den Einspruch: **07.04.2010**
(45) Hinweis auf die Patenterteilung: 25.07.2001
(21) Anmeldenummer: 97925003.2
(22) Anmeldetag: 28.05.1997
(51) Int. Cl.: C07C 7/20, C07C 15/44, C07C 15/46, C07C 51/50

(54) **MISCHUNGEN, ENTHALTEND MONOMERE UND STABILISATOREN**
MIXTURES CONTAINING MONOMERS AND STABILIZERS
MELANGES CONTENANT DES MONOMERES ET DES AGENTS STABILISANTS

(30) Priorität: 05.06.1996 DE 19622498; 27.08.1996 DE 19634470
(43) Veröffentlichungstag der Anmeldung: 07.04.1999
(73) Patentinhaber: BASF SE, 67056 Ludwigshafen (DE)
(72) Erfinder: SUTORIS, Heinz, Friedrich, D-67227 Frankenthal (DE); AUMÜLLER, Alexander, D-67435 Neustadt (DE); UHR, Hermann, D-67227 Frankenthal (DE)
(86) Internationale Anmeldenummer: PCT/EP1997/002758
(87) Internationale Veröffentlichungsnummer: WO 1997/046504

(56) Entgegenhaltungen:
- EP-A2- 0 581 737
- WO-A-96/29311
- US-A- 5 254 760
- R.H. BOUNDRY, R.F. BOYER: 'Styrene. Its Polymers, Copolymers and Derivatives', 1952, BOOK DIVISION, REINHOLD PUBLISHING CORPORATION, NEW YORK, US Artikel 'ACS Monograph Series', Seiten 259 - 262
- Declaration by H.-F. Sutoris und Diagramme
- K. MATYJASZEWSKI, T.P. DAVIS: 'Handbook of Radical Polymerization', 2002, WILEY-INTERSCIENCE, A. JOHN WILEY & SONS, INC. PUBLICATION Seiten 168-172 - 235-237

## Beschreibung

Die vorliegende Erfindung betrifft Mischungen, welche neben vinylgruppenhaltigen Monomeren zur Verhinderung von deren vorzeitiger Polymerisation während der Reinigung oder Destillation noch mindestens eine Nitroxyl- sowie mindestens eine Nitroverbindung enthalten, ein Verfahren zur Reinigung oder Destillation von solchen Monomeren, ohne daß deren vorzeitige Polymerisation stattfindet, sowie die Verwendung von Mischungen, welche Nitroxyl- und Nitroverbindungen enthalten, zur Inhibierung der vorzeitigen Polymerisation von vinylgruppenhaltigen Monomeren.

Es ist bekannt, daß viele ungesättigte Verbindungen bei Temperaturerhöhung zu, in der Regel radikalisch verlaufender, Polymerisation neigen. So müssen beispielsweise vinylaromatische Verbindungen, wie Styrol oder α-Methylstyrol, mit geeigneten Verbindungen stabilisiert werden, um eine vorzeitige Polymerisation bei der destillativen Reinigung der großtechnisch erhaltenen Rohprodukte zu verhindern. Üblicherweise werden dabei diese Stabilisatoren oder Polymerisationsinhibitoren den zu destillierenden Rohprodukten vor dem Reinigungsschritt zugesetzt. Trotz dieser Maßnahme erhält man nach wie vor beträchtliche Anteile an Polymeren. Im Einzelfall kann, besonders auch bei Betriebsstörungen, während der Reinigung oder Destillation eine komplette Polymerisation der vorliegenden Monomeren oder Monomerengemischs erfolgen. Hier resultieren durch den enormen Reinigungsaufwand und den Produktionsausfall hohe Kosten.

In den sowjetischen Patentschriften 1 027 150, 1 558 888 und 1 139 722 wird die Stabilisierung von Styrol durch Verwendung von Nitroxyl- oder Bisnitroxylverbindungen beschrieben.

Aus der japanischen Schrift Hei 1-165 534 sind I-Piperidyloxy-Derivate als Polymerisationsinhibitoren für Styrol bekannt. US-Patent 3 733 326 beschreibt die Inhibierung der Polymerisation von Vinylmonomeren durch Einsatz von radikalischen Precursorverbindungen.

Nitroverbindungen wie 2-Nitro-p-kresol oder 2,6-Dinitro-p-kresol werden in den US-Patentschriften 4 086 147, 4 105 506 und 4 252 615 als Polymerisationsinhibitoren aufgeführt. Die US-Patentschriften 4 132 602 und 4 132 603 offenbaren die Verwendung von halogenierten nitroaromatischen Verbindungen zur Inhibierung der Polymerisation von Vinylaromaten während deren destillativen Aufarbeitung.

Alle diese genannten Nitroverbindungen besitzen jedoch nur geringe Stabilisierungswirkung, weshalb sie in verhältnismäßig hohen Konzentrationen eingesetzt werden müssen. Berücksichtigt man noch die relativ hohe Toxizität dieser Nitroverbindungen, bringt deren Einsatz ein beträchtliches Gefahrenpotential für das Bedienungspersonal sowie die Umwelt mit sich.

Die Verwendung von Inhibitoren auf Basis von N-Nitrosodiphenylamin in Kombination mit Dinitrokresolderivaten bei der Vakuumdestillation von Vinylaromaten wird in den US-Patentschriften 3 988 212 und 4 341 600 beschrieben. Bei Anwesenheit von Sauerstoff läßt sich eine Polymerisation von vinylaromatischen Verbindungen beim Erhitzen nach der Lehre der US-PS 4 466 904 und 4 468 343 durch Verwendung eines Inhibitors auf Basis von Phenothiazin, 4-tert.-Butylcatechol, 2,6-Dinitro-o-kresol oder 2,6-Dinitro-p-kresol mit entweder Phenyldiamin oder 4-tert.-Butylcatechol verhindern. Nach EP 240 297 kann eine Polymerisation von Vinylaromaten beim Erhitzen durch Einsatz von Hydroxylaminderivaten und Dinitrophenol unterbunden werden. Ein Nachteil dieser Inhibitorsysteme besteht jedoch in der starken Abhängigkeit ihrer Wirksamkeit vom Sauerstoffgehalt, d.h. es muß unter den Bedingungen der Reinigung oder Destillation entsprechend der uneinheitlichen Verteilung des Restsauerstoffs in dem entsprechenden Aggregat mit einer unterschiedlichen Inhibierungswirkung dieser Zusätze gerechnet werden. Dies erschwert aber deren kontrollierten Einsatz.

Mischungen von Nitroxyl- und Nitroverbindungen werden in der US-Patentschrift 5 254 760 zur Stabilisierung von vinylaromatischen Verbindungen während der Reinigung oder Destillation beschrieben. Die Nitroxyl- und Nitroverbindung wird dabei in Mengen von 5 bis 95 Gew.-% und 95 bis 5 Gew.-%, bezogen auf die Gesamtmenge der Mischung eingesetzt. Da Nitroxylverbindungen in der Regel sehr teuer sind, stellt dieser Stabilisierungszusatz, der in bestimmtem Anteil dem Rohprodukt vor der Aufarbeitung kontinuierlich zugegeben werden muß, einen nicht zu vernachlässigenden Kostenfaktor dar.

Weiter besteht die Gefahr, daß bei Verwendung von Polymerisationsinhibitoren mit relativ hohen Anteilen an Nitroxylverbindung(en), diese zum Teil in das Reinprodukt verschleppt werden und dort zu einer Hemmung der möglicherweise gewollten Polymerisation führen.

Ein weiterer Nachteil dieser Mischungen ist deren relativ geringe Wirksamkeit im Hinblick auf die zeitliche Verzögerung der Polymerisation der Monomeren. Wird beispielsweise als Folge einer Betriebsstörung die Zuführung der Inhibitor/Rohmonomeren-Mischung in die Destillationsapparatur unterbrochen, so fehlt nicht nur die durch deren kontinuierliche Einleitung bewirkte Kühlung des Kolonnensumpfs, sondern es findet zusätzlich durch unzureichende Stabilisierung eine exotherme Polymerisation mit Freisetzung von Wärme und damit einhergehender Temperaturerhöhung statt, welche wiederum die Polymerisation beschleunigt. Im Extremfall kann so ein entsprechendes Reinigungs- oder Destillationsaggregat durch massive Polymerenbildung für längere Zeit unbrauchbar werden.

Eine hohe zeitliche Retardierung der Polymerisation ist daher für den betrieblichen Einsatz entsprechender Inhibitoren ein wichtiger Gesichtspunkt.

Somit bestand die Aufgabe der vorliegenden Erfindung darin, Mischungen von vinylgruppenhaltigen Monomeren zur Verfügung zu stellen, welche für eine ausreichend lange Zeit wirksam und kostengünstig gegen vorzeitige Polymerisation während der Reinigung oder Destillation stabilisiert sind und im Reinprodukt möglichst keine störenden Inhibitorreste mehr enthalten.

Überraschenderweise wurde gefunden, daß alle diese Kriterien durch Stoffmischungen erfüllt werden, welche
(A) vinylgruppenhaltige Monomere,
(B) eine wirksame Menge einer, die vorzeitige Polymerisation der vinylgruppenhaltigen Monomeren während deren Reinigung oder Destillation inhibierende Mischung, enthaltend,
   (i) 0,05 bis 4,5 Gew.-%, bezogen auf die Gesamtmischung (B), mindestens einer N-Oxyl-Verbindung eines sekundären Amins, welches keine Wasserstoffatome an den α-C-Atomen trägt, sowie
   (ii) 99,95 bis 95,5 Gew.%, bezogen auf die Gesamtmischung (B) mindestens einer aromatischen Nitroverbindung der Formel (III) 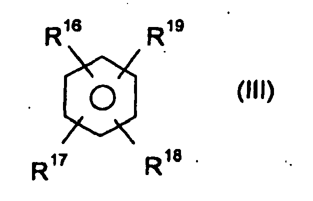 worin
      - R¹⁶, R¹⁷, R¹⁸ und R¹⁹: unabhängig voneinander jeweils Wasserstoff, C₁-C₆-Alkyl, Halogen oder einen Rest der Formel CN, SCN, NCO, OH, NO₂, COOH, CHO, SO₂H oder SO₃H bedeuten,
      mit der Maßgabe, dass mindestens einer der Reste R¹⁶, R¹⁷, R¹⁸ und R¹⁹ jeweils eine Nitrogruppe, eine Hydroxygruppe und eine C₁-C₆-Alkylgruppe ist, und der aromatische Ring zusätzlich'noch benzoanelliert sein kann,
   enthalten.

Bevorzugt sind Stoffmischungen, welche 0,1 bis 4,0 Gew.-% der Komponente (i) und 99,9 bis 96 Gew.-% der Komponente (ii), jeweils bezogen auf die Gesamtmischung (B), enthalten,

Bevorzugte vinylgruppenhaltige Monomere (A) sind solche der Formel (Ia) 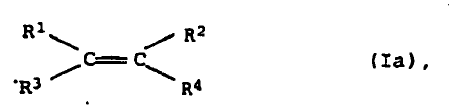 worin bedeuten:
R¹,R²,R³ und R⁴unabhängig voneinander jeweils Wasserstoff, C₁-C₆-Alkyl, C₂-C₆-Alkenyl, unsubstituierte oder substituierte aromatische oder heteroaromatische Reste oder Halogen,
mit der Bedingung, daß nicht mehr als zwei dieser Reste zugleich unsubstituierte oder substituierte aromatische oder heteroaromatische Reste sind.

Die C₁-C₆-Alkylreste umfassen dabei die linearen Alkylketten von Methyl über Ethyl bis zu Hexyl aber auch die entsprechenden verzweigten Reste. Ebenso kommen als C₂-C₆-Alkenylreste Ethenyl, Propenyl usw. bis hinauf zu Hexenyl sowie die im gesättigten Teil verzweigten Gruppen in Frage. Als aromatische oder heteroaromatische sowohl unsubstituierte wie auch substituierte Gruppen sind beispielsweise Phenyl, Pyridyl, Alkylphenyl oder -pyridyl, wie Methylphenyl oder -pyridyl oder Ethylphenyl oder -pyridyl, Alkenylphenyl oder -pyridyl, wie Vinylphenyl oder Vinylpyridyl, Carboxyphenyl oder -pyridyl, Formylphenyl oder -pyridyl, Sulfophenyl oder -pyridyl, Hydroxyphenyl oder -pyridyl, Aminophenyl oder -pyridyl, Nitrophenyl oder -pyridyl aber auch Naphthyl oder mit Alkyl-, Alkenyl-, Carboxy-, Formyl-, Sulfo-, Hydroxy-, Amino-oder Nitro-Gruppen substituiertes Naphthyl zu nennen. Üblicherweise wird als Halogenrest Fluor oder Chlor, gelegentlich auch Brom verwendet.

Werden etwa Verbindungen mit je einem aromatischen oder heteroaromatischen Rest einerseits sowie einem C₁-C₆-Alkyl andererseits in Betracht gezogen, so ergeben sich, wenn die verbleibenden beiden Reste aus R¹, R², R³ und R⁴ Wasserstoff sind, exemplarisch als zuzusetzende Monomere α-Methylstyrol (2-Phenyl-propen-1), die beiden β-Methylstyrolisomeren (cis- und trans-1-Phenyl-propen-1), α-Ethylstyrol (2-Phenyl-buten-1), die beiden β-Ethylstyrolisomeren (cis- und trans-1-Phenyl-buten-1) bis zum α-Hexylstyrol (2-Phenyl-octen-1) oder die beiden β-Hexylstyrolisomeren (cis- und trans-1-Phenyl-octen-1).

Analog ergeben sich unter Verwendung des Pyridyl anstelle des Phenylrestes die Verbindungen 2-Pyridyl-propen-1, cis- und trans-1-Pyridyl-propen-1, 2-Pyridyl-buten-1, cis- und trans-1-Pyridyl-buten-1 bis zu 2-Phenyl-octen-1 und den beiden Isomeren cis-1-Pyridylocten-1 und trans-1-Pyridyl-octen-l. Eingeschlossen sind hier natürlich auch die Isomeren, die sich durch die Stellung des Pyridin-N-Atoms zur die Vinyl- mit der Pyridylgruppe verknüpfenden Bindung unterscheiden. Sind der Phenyl- oder Pyridylrest mit den oben erwähnten Gruppen substituiert, so ergeben sich Verbindungen, wie α-Methylstyrolsulfonsäure (2-Sulfophenyl-propen-1), α-Methylnitrostyrol (2-Nitrophenyl-propen-1), α-Ethyl-styrolsulfonsäure (2-Sulfophenyl-buten-1), α-Ethyl-nitrostyrol (2-Nitrophenyl-buten-1), die pyridylanalogen Monomeren oder die cis/trans-Isomeren der entsprechenden β-substituierten Verbindungen. Selbstverständlich sind auch hier die Isomeren eingeschlossen, die sich durch Stellung des Substituenten am Benzolring relativ zur Phenyl-Vinyl-Bindung oder im Falle des substituierten Pyridinrestes, durch die relative Stellung von Pyridin-N-Atom, Substituent und Pyridyl-Vinyl-Bindung zueinander ergeben.

Durch Wahl eines aromatischen oder heteroaromatischen Restes einerseits sowie eine C₂-C₆-Alkenylgruppe andererseits lassen sich, wenn die beiden verbleibenden Reste wiederum Wasserstoff sind, u.a. auch substituierte Butadiene als Monomere ableiten. Eingesetzt werden können z.B. die Verbindungen 1- oder 2-Phenylbutadien, 1- oder 2-Pyridylbutadien mit dem entsprechenden cis/ trans-Isomeren einerseits sowie im Falle des Pyridylrestes wiederum die Stellungsisomeren bedingt durch die relative Lage von N-Atom zu Pyridyl-Vinyl-Bindung. Auch hier können unterschiedlichste, bereits weiter oben angeführte Substituenten am aromatischen oder heteroaromatischen System auftreten.

Weiter können erfindungsgemäß auch aromatisch oder heteroaromatisch substituierte Ethylene, wie Styrol, Vinylpyridin, Divinylbenzol, Nitrostyrol, Styrolsulfonsäure, Vinyltoluol sowiegegebenenfalls - deren Isomere eingesetzt werden.

Gemäß Formel (Ia) sind bei diesen monosubstituierten Ethylenen drei der Reste R¹, R², R³, R⁴, Wasserstoff und nur eine aromatische oder heteroaromatische gegebenenfalls substituierte Gruppe, d.h. in entsprechender Reihenfolge Phenyl, Pyridyl, Vinylphenyl, Nitrophenyl, Sulfophenyl und Methylphenyl. Falls gewünscht, können auch disubstituierte Ethylene, in welchen zwei oder vier Reste R¹, R², R³, R⁴ Wasserstoff und die übrigen Reste aromatische oder heteroaromatische Gruppen sind, eingesetzt werden. Üblicherweise sind dies symmetrisch substituierte Stilbene, wie 4,4'-Diaminostilben, 4,4'-Dinitrostilben, 4,4'-Dinitrostilben-2,2'-disulfonsäure, 4,4'-Diaminostilben-2,2'-disulfonsäure (Flavonsäure) oder deren cis- oder trans-Isomere. Natürlich kann man auch diejenigen Isomeren einsetzen, welche sich hinsichtlich der Stellung des Substituenten oder der Substituenten im aromatischen oder heteroaromatischen System relativ zur Vinylgruppe voneinander unterscheiden. Gemäß Formel (I) sind in diesen Stilbenen zwei der Reste R¹, R², R³, R⁴ Wasserstoff und die verbleibenden, nicht vicinal angeordneten Reste, welche in diesem Fall auch identisch sind, in entsprechender Abfolge Aminophenyl, Nitrophenyl, Nitrosulfophenyl und Aminosulfophenyl.

Halogenhaltige Monomere, wie Vinylchlorid, Vinylidenchlorid, Vinylfluorid, Vinylbromid sowie Chloropren (2-Chlor-1,3-butadien) können ebenfalls in den beanspruchten Mischungen eingesetzt werden.

Selbstverständlich können nicht nur die vinylgruppenhaltigen Monomeren in Mischung mit ihren Isomeren eingesetzt werden, sondern auch in Mischungen untereinander, wie sie z.B. bei deren Herstellung im Rohprodukt anfallen.

Weiterhin bevorzugte vinylgruppenhaltige Monomere (A) sind solche der Formel (Ib)

CH₂ = CH - Q - Z¹ (Ib),

wobei
Q Sauerstoff oder eine Gruppe -NZ²-, 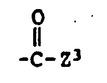 oder -Z³,
Z² Wasserstoff, C₁-C₄-Alkyl oder gemeinsam mit Z³ eine gesättigte oder ungesättigte C₃-, C₄- oder C₅-Alkylenbrücke, in der bis zu zwei Gruppen CH₂ durch NH, N(C₁-C₄-Alkyl), N(C₆-C₁₀-Aryl) oder Sauerstoff und bis zu zwei Gruppen CH durch N ersetzt sein können
   und
Z³ Wasserstoff, C₁-C₄-Alkyl oder einen Rest, der zusammen mit Z² eine gesättigte oder ungesättigte C₃-, C₄- oder C₅-Alkylenbrücke, in der bis zu zwei Gruppen CH₂ durch NH, N(C₁-C₄-Alkyl), N(C₆-C₁₀-Aryl) oder Sauerstoff und bis zu zwei Gruppen CH durch N ersetzt sein können, bedeuten.

Die Monomeren (A) der Formel Ib, die in den erfindungsgemäßen Gemischen enthalten sind, können als Variable Q Sauerstoff enthalten. Von diesen Monomeren eignen sich besonders die Vinylether, in denen Z¹ eine C₁-C₄-Alkylgruppe, also Methyl, Ethyl, n-Propyl, iso-Propyl, n-Butyl, iso-Butyl, sec-Butyl oder tert-Butyl ist, als Bestandteil der erfindungsgemäßen Monomerenzusammensetzungen.

Ist die Variable Q eine Gruppe -NZ²-, so ist Z¹ vorzugsweise eine Gruppe -CO-Z³.

Als Reste Z³ kommen neben Wasserstoff und den genannten C₁-C₄-Alkylgruppen auch solche Reste in Betracht, die zusammen mit der Gruppe -NZ²- einen gesättigten oder ungesättigten 5-bis 7-gliedrigen Ring bilden. Beispiele solcher Ringsysteme sind: 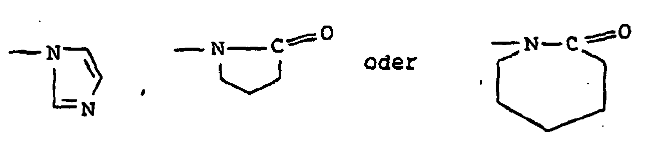 darunter besonders der N-Pyrrolidinonyl- und der N-Caprolactamylrest.

Bevorzugte Monomere in den erfindungsgemäßen Zusammensetzungen sind N-Vinylformamid, N-Vinyl-2-pyrrolidon, N-Vinyl-ε-caprolactam sowie die oben genannten C₁-C₄-Alkylvinylether.

Besonders bevorzugt unter diesen Monomeren ist N-Vinylformamid.

Bevorzugte N-Oxyl-Verbindungen in den erfindungsgemäßen Monomerenzusammensetzungen sind solche der allgemeinen Formel (II) 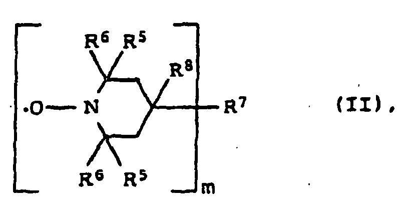 wobei
- R⁵ und R⁶: unabhängig voneinander jeweils C₁-C₄-Alkyl, Phenyl oder gemeinsam mit dem C-Atom, an das sie gebunden sind, einen 5- oder 6-gliedrigen gesättigten Kohlenwasserstoffring,
- R⁷: Wasserstoff, Hydroxy, Amino oder einen m-wertigen über Sauerstoff oder Stickstoff gebundenen organischen Rest oder zusammen mit R⁸ Sauerstoff oder eine unter R⁸ definierte Ringstruktur,
- R⁸: Wasserstoff, C₁-C₁₂-Alkyl oder zusammen mit R⁷ Sauerstoff oder zusammen mit R⁷ und dem C-Atom, an das sie gebunden sind, folgende Ringstrukturen 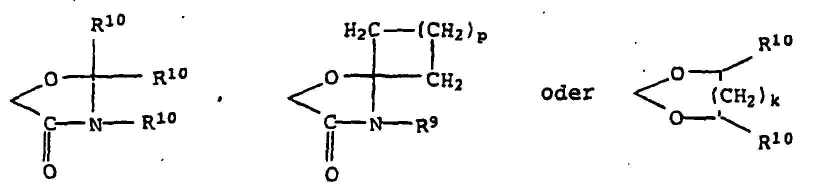
wobei für die Fälle, in denen R⁷ mit R⁸ einen gemeinsamen Rest bildet, m = 1 ist,
- R⁹: Wasserstoff, C₁-C₁₂-Alkyl oder -(CH₂)_{z}-COOR¹⁰,
- R¹⁰: gleiches oder verschiedenes C₁-C₁₈-Alkyl,
- k: 0 oder 1,
- z und p: unabhängig voneinander jeweils 1 bis 12 und
- m: 1 bis 100
bedeuten.

R⁵ und R⁶ können C₁-C₄-Alkylgruppen, wie Methyl, Ethyl, n-Propyl, iso-Propyl, n-Butyl, iso-Butyl, sec-Butyl oder tert.-Butyl sein oder sie können zusammen eine Tetra- oder Pentamethylengruppe bilden. Vorzugsweise sind R⁵ und R⁶ Methylgruppen.

Als R⁸ kommen beispielsweise Wasserstoff, die oben genannten C₁-C₄-Alkylgruppen sowie Pentyl, sec.-Pentyl, tert.-Pentyl, Neopentyl, Hexyl, 2-Methylpentyl, Heptyl, 2-Methylhexyl, Octyl, Isooctyl, 2-Ethylhexyl, Nonyl, 2-Methylnonyl, Isononyl, 2-Methyloctyl, Decyl, Isodecyl, 2-Methylnonyl, Undecyl, Isoundecyl, Dodecyl und Isododecyl, (die Bezeichnungen Isooctyl, Isononyl und Isodecyl sind Trivialbezeichnungen und stammen von den nach der Oxosynthese erhaltenen Carbonylverbindungen ab; vgl. dazu Ullmann's Encyclopedia of Industrial Chemistry, 5th Edition, Vol.

A1, Seiten 290-293, sowie Vol. A10, Seiten 284 und 285) in Betracht.

p ist bevorzugt 6 bis 12, besonders bevorzugt 9.

z ist bevorzugt 1 bis 4, besonders bevorzugt 2.

Als R⁹ kommen neben Wasserstoff beispielsweise die oben angegebenen C₁-C₁₂-Alkylgruppen in Betracht. Bevorzugt steht R⁹ für Wasserstoff, C₁-C₄-Alkyl oder (CH₂)_{z}-COO(C₁-C₆-Alkyl), besonders bevorzugt für die Reste -CH₂-CH₂-COO(CH₂)₁₁-CH₃ und -CH₂-CH₂-COO(CH₂)₁₃-CH₃.

R¹⁰ kann beispielsweise eine der oben genannten C₁-C₁₂-Alkylgruppen oder Tridecyl, Isotridecyl, Tetradecyl, Pentadecyl, Hexadecyl, Heptadecyl oder Octadecyl sein. Bevorzugt sind Dodecyl und Hexadecyl.

Bevorzugte einwertige über Sauerstoff gebundene organische Reste R⁷ sind Hydroxy und (C₁-C₄)-Alkoxygruppen wie beispielsweise Methoxy, Ethoxy, Propoxy und t-Butoxy.

Bevorzugte m-wertige Reste R⁷ sind beispielsweise die folgenden Reste 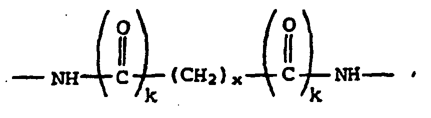 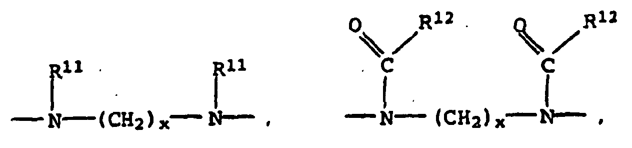 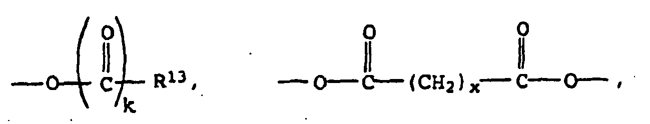 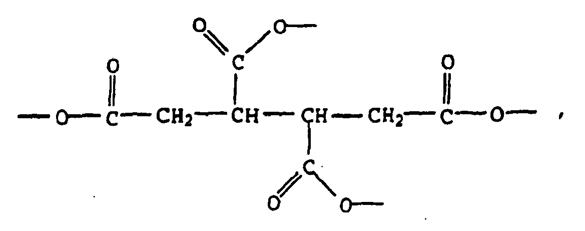 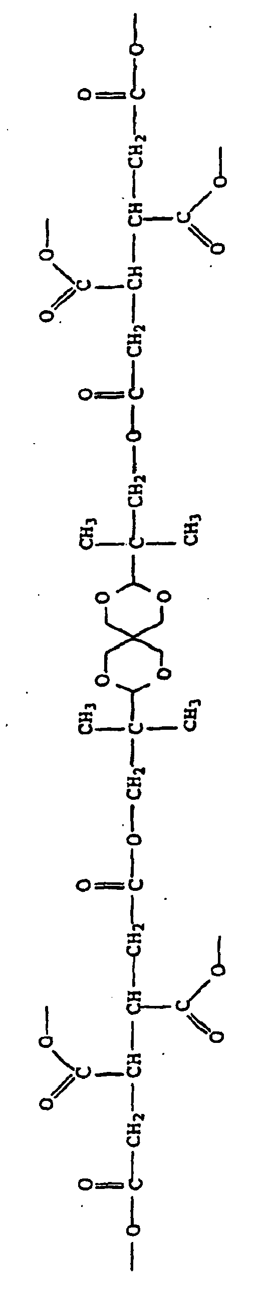 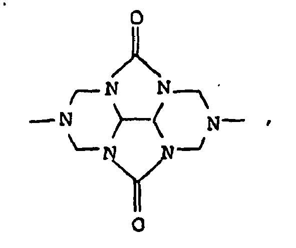 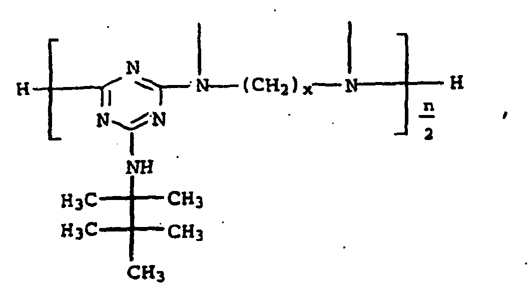 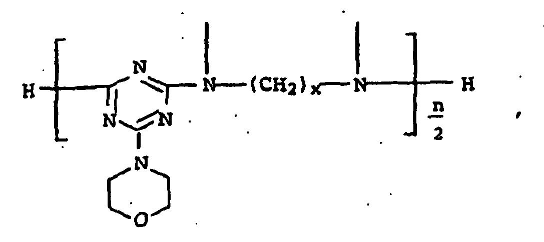 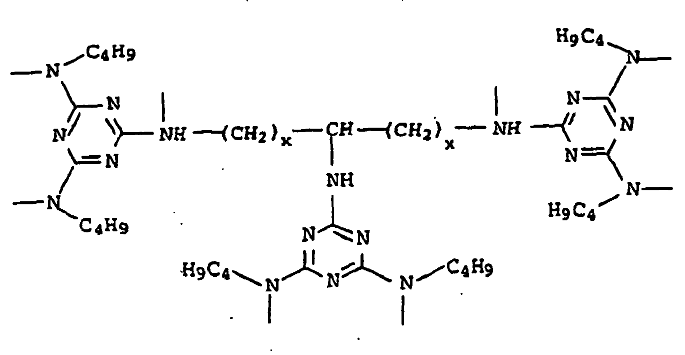 oder 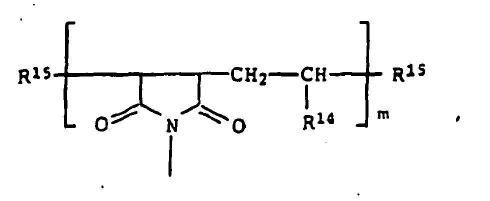 wobei
- R¹¹: C₁-C₁₂-Alkyl oder -(CH₂)_{z}-COOR¹⁰,
- R¹²: Wasserstoff oder C₁-C₁₈-Alkyl,
- R¹³: C₁-C₁₈-Alkyl, Vinyl oder Isopropenyl,
- R¹⁴: C₈-C₂₂-Alkyl,
- R¹⁵: Wasserstoff oder einen organischen Rest, wie er bei der radikalischen Polymerisation der Ausgangsmonomeren üblicherweise entsteht,
- k: 0 oder 1,
- x: 1 bis 12 und
- n: eine gerade Zahl m
bedeuten.

Ist R⁷ einer dieser Reste, so ist R⁸ bevorzugt Wasserstoff. Die Variable m kann dabei 1 bis 100 bedeuten. Bevorzugt ist m 1,2,3,4 oder eine Zahl von 10 bis 50, wobei besonders bei den oligomeren oder polymeren Resten R⁷ in der Regel Gemische eingesetzt werden.

Als R¹¹ kommen die gleichen Reste in Betracht, wie sie für R⁹ genannt sind. Bevorzugt steht R¹¹ für C₁-C₄-Alkyl.

Als R¹² kommen neben Wasserstoff die gleichen Reste in Betracht, wie sie für R¹⁰ genannt worden sind. Bevorzugt steht R¹² für Wasserstoff.

Als R¹³ kommen besonders Vinyl, Isopropenyl oder C₁₅-C₁₇-Alkylreste in Betracht.

Als R¹⁴ kommen beispielsweise die oben genannten C₈-C₁₈-Alkylreste sowie Nonadecyl, Eicosyl, Uneicosyl und Doeicosyl in Betracht. Dabei sind Mischungen verschiedener Reste R¹⁴, die sich in der Länge der Kohlenstoffkette unterscheiden, bevorzugt.

Die Reste R¹⁵ sind Wasserstoff oder organische Reste, wie sie bei der radikalischen Polymerisation der Ausgangsmonomeren entstehen, also z.B. ein Rest, der aus dem Polymerisationsinitiator oder aus einem intermediär aufgetretenen Radikal entsteht oder ein anderer derartiger Rest, wie er dem Fachmann geläufig ist.

Bevorzugte Nitroxylverbindungen als Komponente (i) der erfindungsgemäßen Monomerenzusammensetzungen sind auch die folgenden:
1-Oxyl-2,2,6,6-tetramethylpiperidin,
1-Oxyl-2,2,6,6-tetramethylpiperidin-4-ol,
1-Oxyl-2,2,6,6-tetramethylpiperidin-4-on,
1-Oxyl-2,2,6,6-tetramethylpiperidin-4-yl-acetat,
1-Oxyl-2,2,6,6-tetramethylpiperidin-4-yl-2-ethylhexanoat,
1-Oxyl-2,2,6,6-tetramethylpiperidin-4-yl-stearat,
1-Oxyl-2,2,6,6-tetramethylpiperidin-4-yl-benzoat,
1-Oxyl-2,2,6,6-tetramethylpiperidin-4-yl-(4-tert-butyl)benzoat,
Bis(1-oxyl-2,2,6,6-tetramethylpiperidin-4-yl)-succinat,
Bis(1-oxyl-2,2,6,6-tetramethylpiperidin-4-yl)-adipat,
Bis(1-oxyl-2,2,6,6-tetramethylpiperidin-4-yl)-sebacat,
Bis(1-oxyl-2,2,6,6-tetramethylpiperidin-4-yl)-n-butylmalonat,
Bis(1-oxyl-2,2,6,6-tetramethylpiperidin-4-yl)-phthalat,
Bis(1-oxyl-2,2,6,6-tetramethylpiperidin-4-yl)-isophthalat,
Bis(1-oxyl-2,2,6,6-tetramethylpiperidin-4-yl)-terephthalat,
Bis(1-oxyl-2,2,6,6-tetramethylpiperidin-4-yl)-hexahydroterephthalat,
N,N'-Bis(1-oxyl-2,2,6,6-tetramethylpiperidin-4-yl)-adipinamid,
N-(1-Oxyl-2,2,6,6-tetramethylpiperidin-4-yl)-caprolactam,
N-(1-Oxyl-2,2,6,6-tetramethylpiperidin-4-yl)-dodecylsuccinimid,
2,4,6-Tris-[N-butyl-N-(1-oxyl-2,2,6,6,-tetramethylpiperidin-4-yl]-s-triazin,
4,4'-Ethylenbis(l-oxyl-2,2,6,6-tetramethylpiperazin-3-on),
N,N'-Bis(1-oxyl-2,2,6,6-tetramethylpiperidin-4-yl)-N,N'-bis-formyl-1,6-diaminohexan und Tris-(2,2,6,6-tetramethyl-1-oxylpiperidin-4-yl)phosphit.

Die beschriebenen Nitroxylverbindungen lassen sich aus den entsprechenden Piperidinverbindungen durch Oxidation z.B. mit Wasserstoffperoxid herstellen. Details zu dieser Oxidation sind z.B. in der älteren deutschen Patentanmeldung 195 101 84.7 genannt. Die sekundären Amine, welche an den α-C-Atomen keine Wasserstoffatome tragen, wie Piperidinverbindungen, und ihre Herstellung sind allgemein bekannt. Da die Oxidationsreaktionen nicht immer vollständig ablaufen, können auch die als Ausgangsverbindungen dienenden Piperidinverbindungen sowie teilweise oxidierte Zwischenstufen in den erfindungsgemäßen Monomerenzusammensetzungen enthalten sein.

In Frage kommen aromatische Nitroverbindungen, wie 2,6-Dinitro-4-methylphenol, 2-Nitro-4-methylphenol, 2,4-Dinitro-6-sec-butylphenol bzw. 2,4-Dinitro-6-methylphenol verwendet, in welchen je einer der Reste R¹⁶, R¹⁷, R¹⁸ und R¹⁹ eine Nitro-, eine Hydroxy- und eine C₁-C₆-Alkylgruppe ist.

Weiter kann der Mischung (B) zusätzlich zu den Komponenten (i) und (ii) noch ein oder mehrere Costabilisatoren aus der Gruppe der aromatischen Nitrosoverbindungen, Phenothiazine, Chinone, Hydrochinone und deren Ether, Phenole und deren Ether, Hydroxylamine und Phenylendiamine, zugesetzt werden.

Als aromatische Nitrosoverbindungen kommen z.B. p-Nitrosophenol, p-Nitroso-o-kresol oder p-Nitroso-N,N'-diethylanilin in Betracht.

Weitere Costabilisatoren können auch substituierte Phenole oder Hydrochinone, beispielsweise die folgenden:
4-tert-Butylbrenzcatechin, Methoxyhydrochinon, 2,6-Di-tert-butyl-4-methylphenol, n-Octadecyl-β-(3,5-di-tert-butyl-4-hydroxyphenyl)-propionat, 1,1,3-Tris-(2-methyl-4-hydroxy-5-tert-butyl-phenyl)-butan, 1,3,5-Trimethyl-2,4,6-tris-(3,5-di-tert-butyl-4-hydroxybenzyl)-benzol, 1,3,5-Tris-(3,5-di-tert-butyl-4-hydroxybenzyl)-isocyanurat, 1,3,5-Tris-[β-(3,5-di-tert-butyl-4-hydroxyphenyl)-propionyloxyethyl]-isocyanurat, 1,3,5-Tris-(2,6-dimethyl-3-hydroxy-4-tert-butylbenzyl)-isocyanurat oder Pentaerythrittetrakis-[β-(3,5-di-tert-butyl-4-hydroxy-phenyl)-propionat] sein.

Zur Stabilisierung der erfindungsgemäßen Monomerenzusammensetzungen enthalten diese Zusammensetzungen eine wirksame Menge der Mischung (B), was im allgemeinen 0,0002 bis 5 Gew.-%, vorzugsweise 0,0005 bis 0,5 Gew.-%, der Mischung (B), jeweils bezogen auf die Gesamtmenge der Monomerenzusammensetzung bedeutet.

Selbstverständlich können die Mischungen (B) auch Mischungen von verschiedenen erfindungsgemäßen 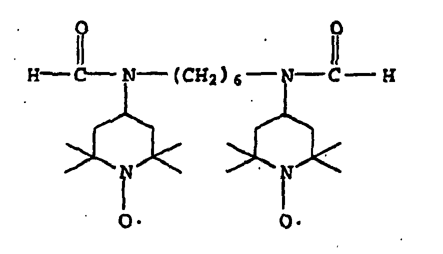 Nitroxyl- und Nitroverbindungen sowie der genannten Costabilisatoren enthalten.

Mischung (B) läßt sich den Monomeren vor oder während der Reinigung oder Destillation in einer wirksamen Menge zugeben, um eine vorzeitige Polymerisation zu unterbinden. Im speziellen Fall kann es auch erforderlich sein, die Komponenten (i) und (ii), gegebenenfalls unter Zugabe eines oder mehrerer der genannten Costabilisatoren, getrennt und dann bevorzugt an räumlich unterschiedlichen Stellen zuzusetzen.

Die erfindungsgemäßen Stabilisatormischungen (B) können entweder in Substanz oder als Suspension oder Lösung unter Zuhilfenahme von geeigneten Verdünnungsmitteln angewandt werden. Sie können generell zur Inhibierung der vorzeitigen Polymerisation von vorzugsweise radikalisch polymerisierbaren Monomeren verwendet werden und zeigen ihre stabilisierende Wirkung in einem breiten Temperaturbereich. Sie sind bei jeder üblichen Lagertemperatur von -50 bis +50°C wirksam und ebenso bei erhöhten Temperaturen, wie sie beispielsweise bei der Destillation oder Reinigung der Monomeren angewendet werden. Auch der Druckbereich des Stabilisierungsverfahrens ist unkritisch. Die Stabilisatoren wirken bei Normaldruck und auch bei vermindertem Druck, wie er teilweise bei Destillationsprozessen angewendet wird.

### Beispiele

### 1. Herstellung von

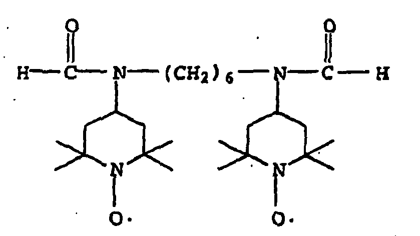

Eine Suspension aus 540 g (1,37 mol) N,N'-Bis-[2,2,6,6-tetramethylpiperidin-4-yl]-N,N'-bis-formyl-1,6-diaminohexen, 800 ml Wasser, 150 ml Isobutanol und 200 mg Magnesiumsulfat wurde bei 70°C im Laufe von 2 h mit 600 ml einer 30 gew.-%igen wäßrigen Lösung von Wasserstoffperoxid (19,6 mol) versetzt und anschließend noch 16 h bei dieser Temperatur gehalten. Danach wurde die Mischung auf Raumtemperatur abgekühlt und das ausgefallene Produkt wie üblich isoliert.

Ausbeute: 85 %, Schmelzpunkt: 169 bis 170°C.

Charakterisierungen zeigen, daß das erhaltene und für weitere Versuche eingesetzte Produkt (im folgenden F genannt) zu etwa 60 % die Di-Nitroxylverbindung obiger Formel enthält.

### 2. Mischungen:

### Stationäre Messungen:

500 g der in nachfolgender Tabelle 1 aufgeführten Mischungen aus Styrol, dem Produkt F und der Nitroverbindung 2,4-Dinitro-6-sec-butylphenol (DNBP) wurden in einem Reaktionsgefäß unter Stickstoff und Normaldruck auf 110°C erhitzt. In diese temperierte Mischung wurden 250 g pro Stunde einer identischen Mischung kontinuierlich zudosiert und die gleiche Menge kontinuierlich entnommen. Im Auslaß wurde der Gleichgewichtspolymergehalt im stationären Zustand gemessen. Nach 360 min wurde ein Stromausfall simuliert (Batch-Betrieb). Die Zudosierung des Inhibitors wurde gestoppt. Die Temperatur wurde innerhalb von 60 min kontinuierlich auf 145°C erhöht. Der Polymergehalt wurde in Abständen von jeweils 30 min gemessen. Folgende Ergebnisse wurden festgestellt.

**Tabelle 1**

| Mischung | Stabilisator (Mischung (B)) | | | | Monomer (A) | Stabilisatorgehalt in Gesamtmischung | Polymergehalt im stationären Zustand | Polymergehalt nach 60 min Balch-Betrieb |
|---|---|---|---|---|---|---|---|---|
| | Nitroxylverbindung | Gew.-% | Nitroverbindung | Gew.-% | vinylgruppenhaltiges Monomer | | | |
| 1 | F | 2 | DNBP | 98 | Styrol | 0,15 | 0,02 | 0,2 |
| 2 | F | 2,9 | DNBP | 97,1 | Styrol | 0,155 | 0,02 | 0,2 |
| 3 | F | 3 | DNBP | 97 | Styrol | 0,10 | 0,04 | 0,6 |
| 4 | F | 1,5 | DNBP | 98,5 | Styrol | 0,20 | - | - |
| Vergleich | F | 5 | DNBP | 95 | Styrol | 0,06 | 0,08 | > 1 |

### Nichtstationäre Messungen:

In einem 250-ml-Rundkolben, welcher mit Rührer, Rückflußkühler und Innenthermometer ausgestattet war, wurden die in Tabelle 2 aufgeführten Mischungen unter Stickstoffatmosphäre mittels Ölbad auf 140°C erhitzt und bei dieser Temperatur gehalten.

Mit Erreichen der Endtemperatur von 140°C wurde in zeitlichen Intervallen von 15 min (tₒ= 0 min, ist der Zeitpunkt des Erreichens der Endtemperatur) der Polymergehalt bestimmt.

Die Resultate sind in Tabelle 2 wiedergegeben.

**Tabelle 2**

| Mischung | Zeitliche Veränderung des Polymergehalts (%) | | | | | | |
|---|---|---|---|---|---|---|---|
| | 0 min | 15 min | 30 min | 45 min | 60 min | 75 min | 90 min |
| 1 | 0,08 | 0.2 | 0,4 | 0,6 | 1,0 | >2 | - |
| 3 | 0,1 | 0,2 | 0,8 | 1,0 | 2,0 | - | - |
| 4 | 0,06 | 0,1 | 0,2 | 0,4 | 0,8 | 1 | 2 |
| Vergleich | 0,2 | 1 | >2 | - | - | - | - |

## Patentansprüche

1. Stoffmischungen enthaltend
(A) vinylgruppenhaltige Monomere,
(B) eine wirksame Menge einer, die vorzeitige Polymerisation der vinylgruppenhaltigen Monomeren während deren Reinigung oder Destillation inhibierende Mischung, enthaltend,
(i) 0,05 bis 4,5 Gew.-%, bezogen auf die Gesamtmischung (B), mindestens einer N-Oxyl-Verbindung eines sekundären Amins, welches keine Wasserstoffatome an den α-C-Atomen trägt, sowie
(ii) 99,95 bis 95,5 Gew.-%, bezogen auf die Gesamtmischung (B), mindestens eine aromatische Nitroverbindung der Formel (III) 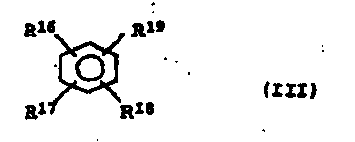 worin
R¹⁶, R¹⁷ ,R¹⁸ und R¹⁹ unabhängig voneinander jeweils Wasserstoff, C₁-C₆-Alkyl, Halogen oder einen Rest der Formel CN, SCN, NCO, OH, NO₂, COOH, CHO, SO₂H oder SO₃H bedeuten,
mit der Maßgabe, daß mindestens einer der Reste R¹⁶, R¹⁷, R¹⁸ und R¹⁹ jeweils eine Nitrogruppe, eine Hydroxygruppe und eine C₁-C₆-Alkylgruppe ist, und der aromatische Ring zusätzlich noch benzoanelliert sein kann.

2. Stoffmischungen nach Anspruch 1, enthaltend 0,1 bis 4,0 Gew.-% der Komponente (i) und 99,9 bis 96,0 Gew.-% der Komponente (ii), jeweils bezogen auf die Gesamtmischung (B).

3. Stoffmischungen nach den Ansprüchen 1 oder 2, welche als vinylgruppenhaltige Monomere (A) Verbindungen der Formel (Ia) 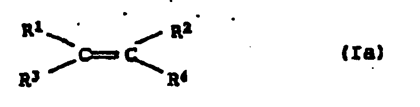 enthalten und worin bedeuten:
R¹,R²,R³ und R⁴ unabhängig voneinander jeweils Wasserstoff, C₁-C₆-Alkyl, C₂-C₆-Alkenyl, unsubstituierte oder sub- stituierte aromatische oder heteroaromatische Reste oder Halogen,
mit der Bedingung, daß nicht mehr als zwei dieser Reste zugleich unsubstituierte oder substituierte aromatische oder heteroaromatische Reste sind.

4. Stoffmischungen nach den Ansprüchen 1 oder 2, welche als vinylgruppenhaltige Monomere (A) Verbindungen der Formel (Ib)
CH₂ = CH - Q - Z¹ (Ib)
enthalten, wobei
Q Sauerstoff oder eine Gruppe -NZ²-,
Z¹ 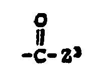 oder -Z³,
Z² Wasserstoff, C₁-C₄-Alkyl oder gemeinsam mit Z³ eine gesättigte oder ungesättigte C₃-, C₄- oder C₅-Alkylenbrücke, in der bis zu zwei Gruppen CH₂ durch NH, N(C₁-C₄-Alkyl), N(C₆-C₁₀-Aryl) oder Sauerstoff und bis zu zwei Gruppen CH durch N ersetzt sein können
und
Z³ Wasserstoff, C₁-C₄-Alkyl oder einen Rest, der zusammen mit Z² eine gesättigte oder ungesättigte C₃-, C₄- oder C₅-Alkylenbrücke, in der bis zu zwei Gruppen CH₂ durch NH, N(C₁-C₄-Alkyl), N(C₆-C₁₀-Aryl) oder Sauerstoff und bis zu zwei Gruppen CH durch N ersetzt sein können, bedeuten.

5. Stoffmischungen nach den Ansprüchen 1 bis 4, welche als Komponente (i) mindestens eine Verbindung der Formel (II) 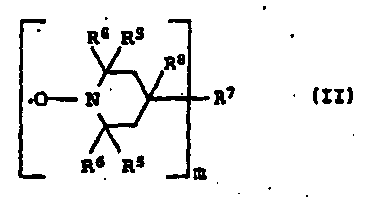 aufweisen, worin
R⁵ und R⁶ unabhängig voneinander jeweils C₁-C₄-Alkyl, Phenyl oder gemeinsam mit dem C-Atom, an das sie gebunden sind, einen 5- oder 6-gliedrigen gesättigten Kohlenwasserstoffring,
R⁷ Wasserstoff, Hydroxy, Amino oder einen m-wertigen über Sauerstoff oder Stickstoff gebundenen organischen Rest oder zusammen mit R⁸ Sauerstoff oder eine unter R⁸ definierte Ringstruktur,
R⁸ Wasserstoff, C₁-C₁₂-Alkyl oder zusammen mit R⁷ Sauerstoff oder zusammen mit R⁷ und dem C-Atom, an das sie gebunden sind, folgende Ringstrukturen 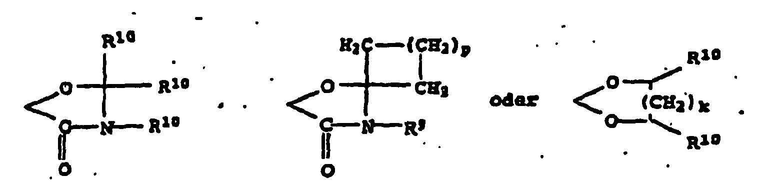
wobei für die Fälle, in denen R⁷ mit R⁸ einen gemeinsamen Rest bildet, m = 1 ist,
R⁹ Wasserstoff, C₁-C₁₂-Alkyl oder -(CH₂)_{z}-COOR¹⁰,
R¹⁰ gleiches oder verschiedenes C₁-C₁₈-Alkyl,
k 0 oder 1,
z und p unabhängig voneinander jeweils 1 bis 12 und
m 1 bis 100
bedeuten.

6. Stoffmischungen nach Anspruch 5, worin R⁷ in Formel (II) ein Rest der Formel
-O(C₁-C₄-Alkyl), 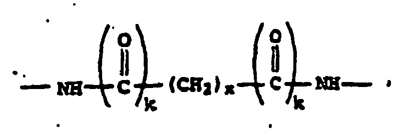 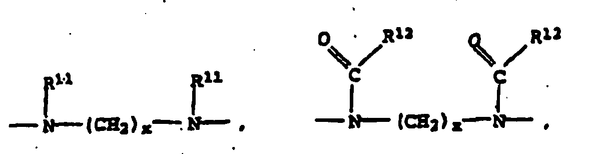 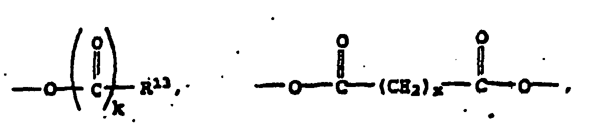 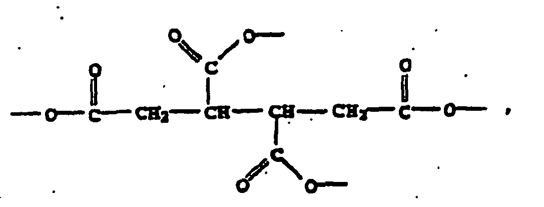 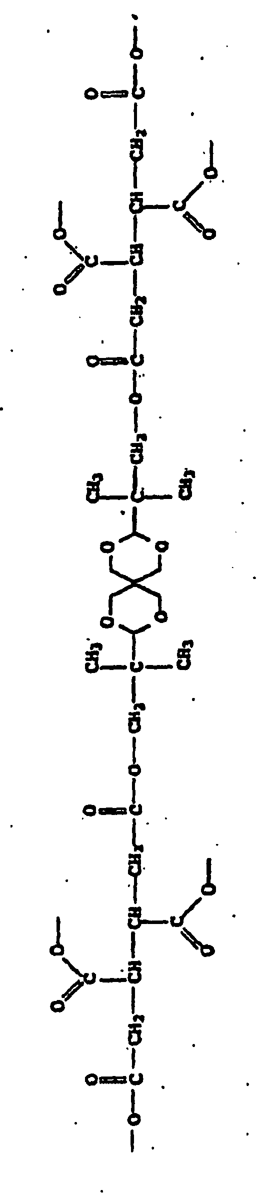 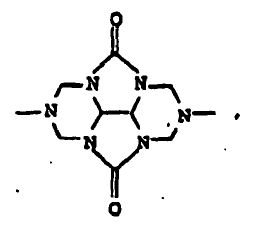 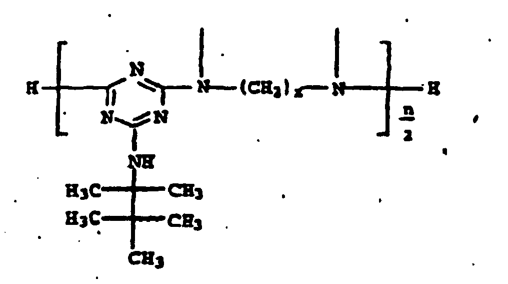 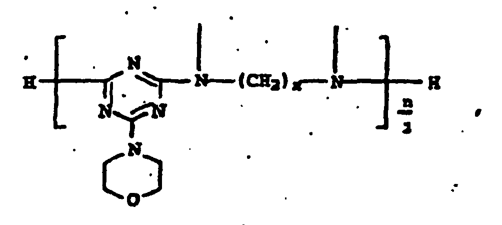 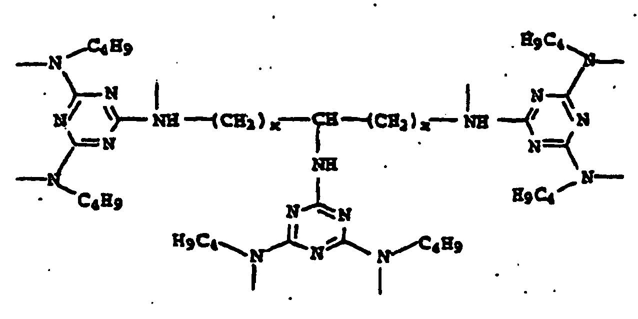
oder 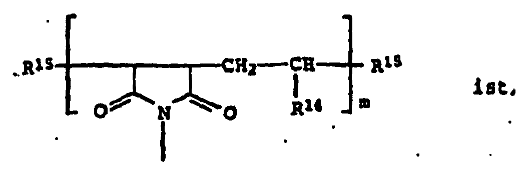 wobei
R¹¹ C₁-C₁₂-Alkyl oder -(CH₂)_{z}-COOR¹⁰,
R¹² Wasserstoff oder C₁-C₁₈-Alkyl,
R¹³ C₁-C₁₈-Alkyl, Vinyl oder Isopropenyl,
R¹⁴ C₈-C₂₂-Alkyl,
R¹⁵ Wasserstoff oder einen organischen Rest, wie er bei der radikalischen Polymerisation der Ausgangsmonomeren (A) üblicherweise entsteht,
k 0 oder 1,
x 1 bis 12 und
n eine gerade Zahl m
bedeuten.

7. Stoffmischung (B) nach den Ansprüchen 1, 2, 5 bis 6, welche zusätzlich zu den Komponenten (i) und (ii) noch einen oder mehrere Costabilisatoren aus der Gruppe der aromatischen Nitrosoverbindungen, Phenothiazine, Chinone, Hydrochinone und deren Ether, Phenole und deren Ether, Hydroxylamine und Phenylendiamine enthalten.

8. Verfahren zur Inhibierung der vorzeitigen Polymerisation von vinylgruppenhaltigen Monomeren während deren Reinigung oder Destillation, **dadurch gekennzeichnet, daß** man den Monomeren vor oder während der Reinigung oder Destillation eine Mischung (B) gemäß den Ansprüchen 1, 2, 5 bis 7 in einer wirksamen Menge zusetzt.

9. Verfahren nachAnspruch 8,
**dadurch gekennzeichnet, daß** man den Monomeren vor der Reinigung oder Destillation die Komponenten der Mischung (B) gemäß den Ansprüchen 1, 2, 5 bis 7 einzeln in jeweils wirksamer Menge zusetzt.

10. Verfahren nach Anspruch 9, **dadurch gekennzeichnet, daß** man die Komponenten an räumlich unterschiedlichen Stellen zusetzt.

11. Verwendung von Mischung (B) nach den Ansprüchen 1, 2, 5 bis 7 zur Inhibierung der vorzeitigen Polymerisation von radikalisch polymerisierbaren Monomeren.

## Claims

1. A mixture comprising
(A) vinyl-containing monomers,
(B) an effective amount of a mixture inhibiting the premature polymerization of the vinyl-containing monomers during their purification or distillation and comprising,
(i) from 0.05 to 4.5 % by weight, based on the total mixture (B), of at least one N-oxyl compound of a secondary amine which carries no hydrogen atoms on the a-carbon atoms and
(ii) from 99.95 to 95.5 % by weight, based on the total mixture (B), of at least one aromatic nitro compound of the formula (III) 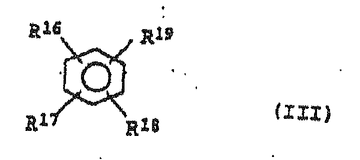 wherein
R¹⁶, R¹⁷, R¹⁸ and R¹⁹, independently of one another, are each hydrogen, C₁-C₆-alkyl, halogen or a radical of the formula CN, SCN, NCO, OH, NO₂, COOH, CHO, SO₂H or SO₃H,
with the proviso that at least one of the radicals R¹⁶, R¹⁷, R¹⁸ and R¹⁹ is nitro, one is hydroxyl and one is C₁-C₆-alkyl and the aromatic ring may additionally be benzofused.

2. The mixture according to claim 1, comprising from 0.1 to 4.0 % by weight of component (i) and from 99.9 to 96.0 % by weight of component (ii), based in each case on the total mixture (B).

3. The mixture according to claim 1 or 2, which comprises, as vinyl-containing monomers (A), compounds of the formula (Ia) 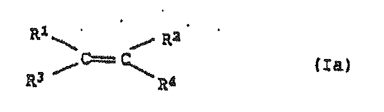 where:
R¹, R², R³ and R⁴, independently of one another, are each hydrogen, C₁-C₆-alkyl, C₂-C₆-alkenyl, unsubstituted or substituted aromatic or heteroaromatic radicals or halogen,
with the proviso that not more than two or these radicals are simultaneously unsubstituted or substituted aromatic or heteroaromatic radicals.

4. The mixture according to claim 1 or 2, which comprises, as vinyl-containing monomers (A), compounds of the formula (Ib)
CH₂ = CH - Q - Z¹ (Ib)
where
Q is oxygen or -NZ²-,
Z¹ is 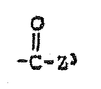
or -Z³,
Z² is hydrogen, or C₁-C₄-alkyl or, together with Z³, is a saturated or unsaturated C₃-, C₄- or C₅-alkylene bridge in which one or two CH₂ groups may be replaced by NH, N(C₁-C₄-alkyl), N(C₆-C₁₀-aryl) or oxygen and one or two CH groups may be replaced by N
and
Z³ is hydrogen, C₁-C₄-alkyl or a radical, which, together with Z², is a saturated or unsaturated C₃-, C₄- or C₅-alkylene bridge in which one or two CH₂ groups may be replaced by NH, N(C₁-C₄-alkyl), N(C₆- C₁₀-aryl) or oxygen and one or two CH groups may be replaced by N.

5. The mixture according to any of claims 1 to 4, which coms, as component (i), at least one compound of the formula (II) 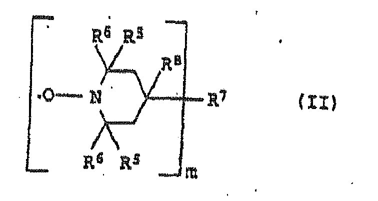 wherein
R⁵ and R⁶, independently of one another, are each C₁-C₄- alkyl or phenyl or, together with the carbon atom to which they are bonded, are a 5- or 6-membered saturated hydrocarbon ring,
R⁷ is hydrogen, hydroxyl, amino or an m-valent organic radical bonded via oxygen or nitrogen or, together with R⁸, is oxygen or a ring structure defined under R⁸,
R⁸ is hydrogen or C₁-C₁₂-alkyl, or together with R⁷ is oxygen or, together with R⁷ and the carbon atom to which they are bonded, forms the following ring structures 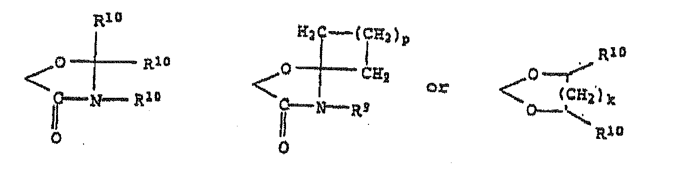 where, when R⁷ together with R⁸ forms a common radical, m is 1,
R⁹ is hydrogen, C₁-C₁₂-alkyl or -(CH₂)_{z}-COOR¹⁰,
R¹⁰ are identical or different C₁-C₁₈-alkyl radicals,
k is 0 or 1,
z and p, independently of one another, are each from 1 to 12 and
m is from 1 to 100.

6. The mixture according to claim 5, wherein R⁷ in the formula (II) is a radical of the formula
-O(C₁-C₄-alkyl), 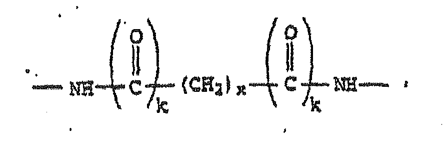 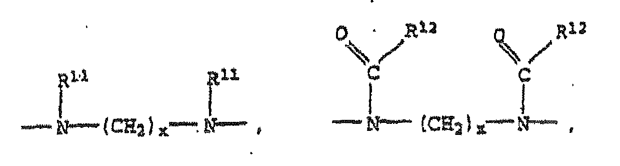 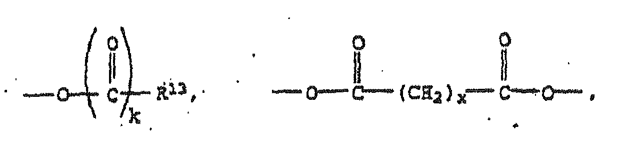 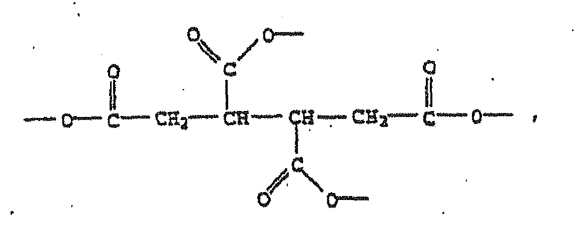 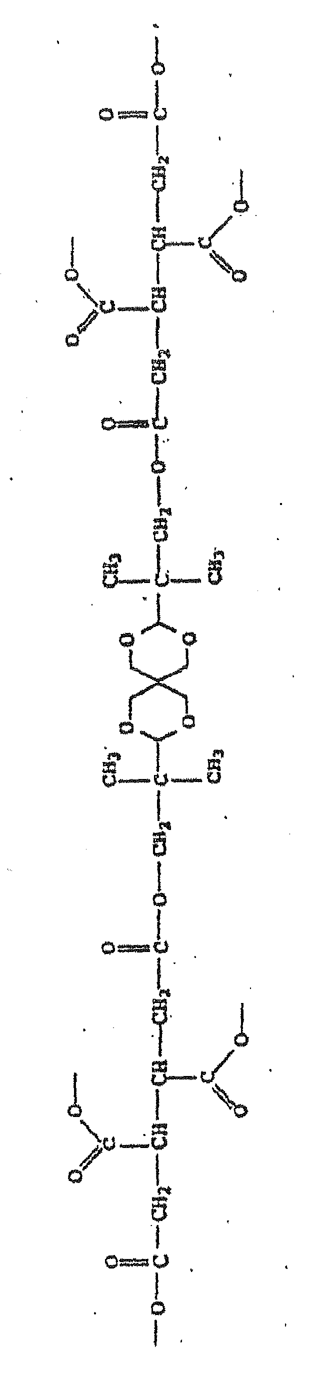 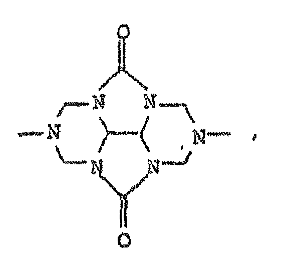 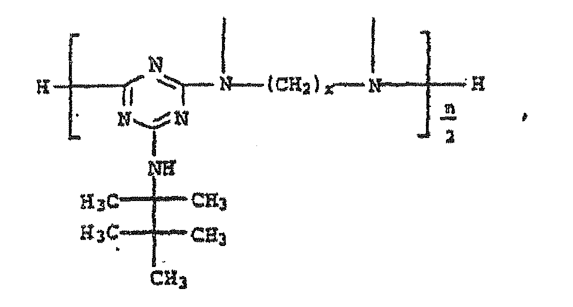 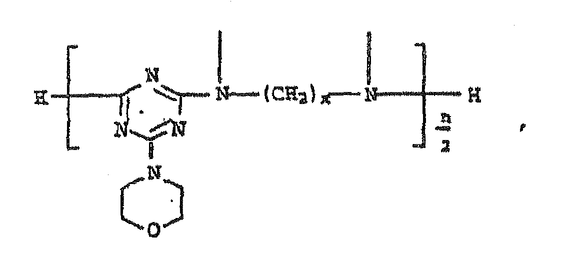 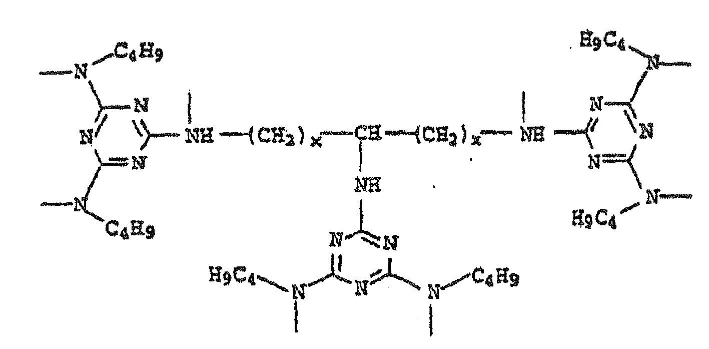
or 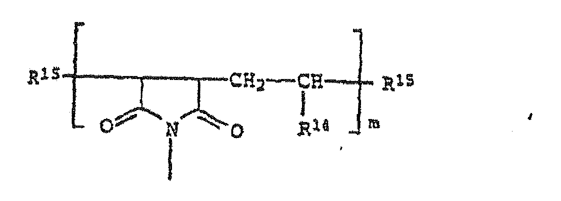 wherein
R¹¹ is C₁-C₁₂-alkyl or -(CH₂)_{z}-COOR¹⁰,
R¹² is hydrogen or C₁-C₁₈-alkyl,
R¹³ is C₁-C₁₈-alkyl, vinyl or isopropenyl,
R¹⁴ is C₈-C₂₂-alkyl,
R¹⁵ is hydrogen or an organic radical as usually formed in the free radical polymerization of the starting monomers (A),
k is 0 or 1,
x is from 1 to 12 and
n is an even number m.

7. The mixture (B) according to any of claims 1, 2, 5 to 6, which also comprises one or more costabilizers selected from the group consisting of the aromatic nitroso compounds, phenothiazines, quinones, hydroquinones and their ethers, phenols and their ethers, hydroxylamines and phenylenediamines, in addition to the components (i) and (ii).

8. A process for inhibiting the premature polymerization of vinyl-containing monomers during their purification or distillation, wherein an effective amount of a mixture (B) according to any of claims 1, 2, 5 to 7 is added to the monomers before or during the purification or distillation.

9. The process according to claim 8, wherein the components of the mixture (B) according to any of claims 1, 2, 5 to 7 are added individually, each in an effective amount, to the monomers before the purification or distillation.

10. The process according to claim 9, wherein the components are added at different points in space.

11. The use of a mixture (B) according to any of claims 1, 2, 5 to 7 for inhibiting the premature polymerization of monomers capable of free radical polymerization.

## Revendications

1. Mélanges de substances contenant
(A) des monomères contenant des groupes vinyles,
(B) une quantité efficace d'un mélange empêchant la polymérisation précoce des monomères contenant des groupes vinyle pendant leur purification ou distillation, contenant,
(i) 0,05 à 4,5% en poids, par rapport au mélange total (B), d'au moins un composé N-oxyle d'une amine secondaire, qui ne porte aucun atome d'hydrogène sur les atomes de C en position a, ainsi que
(ii) 99,95 à 95,5% en poids, par rapport au mélange total (B), d'au moins un composé nitro aromatique de formule (III) 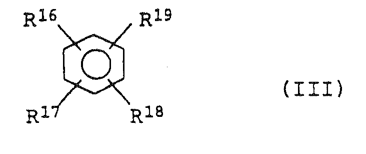 dans laquelle
R¹⁶, R¹⁷, R¹⁸ et R¹⁹ représentent chacun indépendamment les uns des autres, un atome d'hydrogène, un groupe alkyle en C₁ à C₆, un atome d'halogène ou un résidu de formule CN, SCN, NCO, OH, NO₂, COOH, CHO, SO₂H ou SO₃H,
avec la condition qu'au moins un des résidus R¹⁶, R¹⁷, R¹⁸ et R¹⁹ soit respectivement un groupe nitro, un groupe hydroxy, et un groupe alkyle en C₁ à C₆, et le noyau aromatique peut en plus être benzocondensé.

2. Mélanges de substances selon la revendication 1, contenant 0,1 à 4,0% en poids du composant (i) et 99,9 à 96,0% en poids du composant (ii), à chaque fois par rapport au mélange total (B).

3. Mélanges de substances selon les revendications 1 ou 2, qui contiennent en tant que monomères contenant des groupes vinyle (A), des composés de formule (Ia) 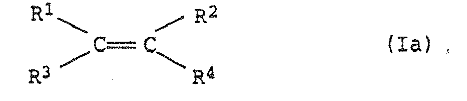 et dans laquelle :
R¹, R², R³ et R⁴ représentent chacun indépendamment les uns des autres, un atome d'hydrogène, un groupe alkyle en C₁ à C₆, alcényle en C₂ à C₆, des résidus aromatiques ou hétéro-aromatiques substitués ou non substitués ou un atome d'halogène,
avec la condition que, pas plus de deux de ces résidus ne soient à la fois des résidus aromatiques ou hétéro-aromatiques non substitués ou substitués.

4. Mélanges de substances selon les revendications 1 ou 2, lesquels contiennent en tant que monomères contenant des groupes vinyle (A), des composés de formule (Ib)
CH₂ = CH - Q - Z¹ (Ib)
où
Q représente un atome d'oxygène ou un groupe -NZ²-,
Z¹ représente 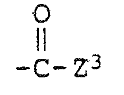 ou -Z³,
Z² représente un atome d'hydrogène, un groupe alkyle en C₁ à C₄, ou conjointement avec Z³, un pont alkylène en C₃, C₄ ou C₅, insaturé ou saturé, dans lequel jusqu'à deux groupes CH₂ peuvent être remplacés par un groupe NH, N-(alkyle en C₁ à C₄), N-(aryle en C₆ à C₁₀) ou un atome d'oxygène et jusqu'à deux groupes CH peuvent être remplacés par N, et
Z³ représente un atome d'hydrogène, un groupe alkyle en C₁ à C₄ ou un résidu, qui représente conjointement avec Z², un pont alkylène en C₃, C₄ ou C₅, saturé ou insaturé, dans lequel jusqu'à deux groupes CH₂ peuvent être remplacés par un groupe NH, N-(alkyle en C₁ à C₄), N-(aryle en C₆ à C₁₀) ou un atome d'oxygène et jusqu'à deux groupes CH peuvent être remplacés par N.

5. Mélanges de substances selon les revendications 1 à 4, lesquels comprennent en tant que composant (i), au moins un composé de formule (II) 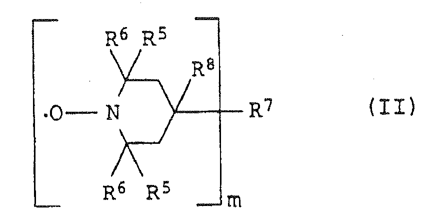 dans laquelle,
R⁵ et R⁶ représentent chacun, indépendamment l'un de l'autre, un groupe alkyle en C₁ à C₄, phényle ou représentent ensemble avec l'atome de C auquel ils sont liés, un cycle hydrocarboné saturé à 5 ou 6 maillons,
R⁷ représente un atome d'hydrogène, un groupe hydroxy, amino ou un résidu organique de valence m lié par l'intermédiaire d'un atome d'oxygène ou d'azote ou représente conjointement avec R⁸ un atome d'oxygène ou une structure cyclique définie sous R⁸,
R⁸ représente un atome d'hydrogène, un groupe alkyle en C₁ à C₁₂, ou représente conjointement avec R⁷ un atome d'oxygène, ou conjointement avec R⁷ et l'atome de C, auquel ils sont liés, les structures cycliques suivantes 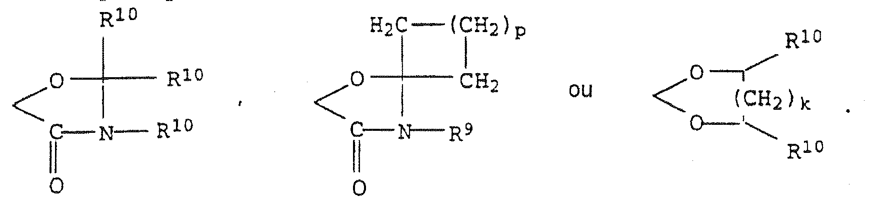 dans lesquelles, pour les cas où R⁷ à R⁸ forment un résidu commun, m = 1,
R⁹ représente un atome d'hydrogène, un groupe alkyle en C₁ à C₁₂, ou -(CH₂)_{z}-COOR¹⁰,
R¹⁰ représente un groupe alkyle en C₁ à C₁₈, identique ou différent,
k vaut 0 ou 1,
z et p valent chacun indépendamment l'un de l'autre, 1 à 12 et
m vaut de 1 à 100.

6. Mélanges de substances selon la revendication 5, dans lesquels R⁷ représente dans la formule (II), un résidu de formule
-O-(alkyle en C₁ à C₄), 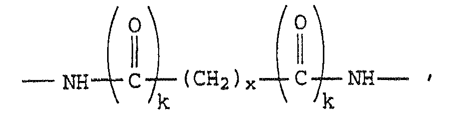 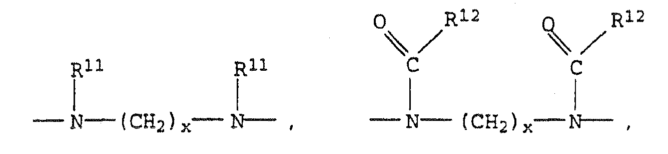 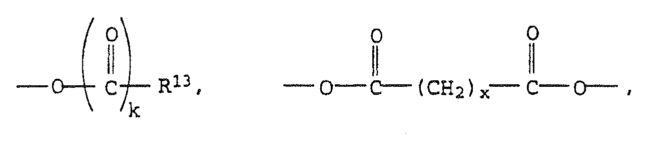 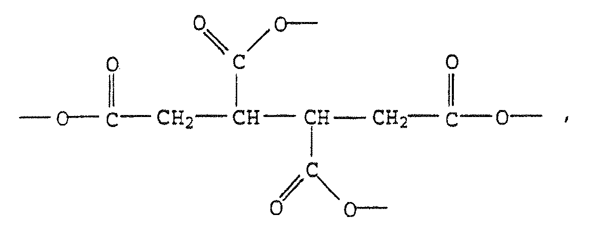 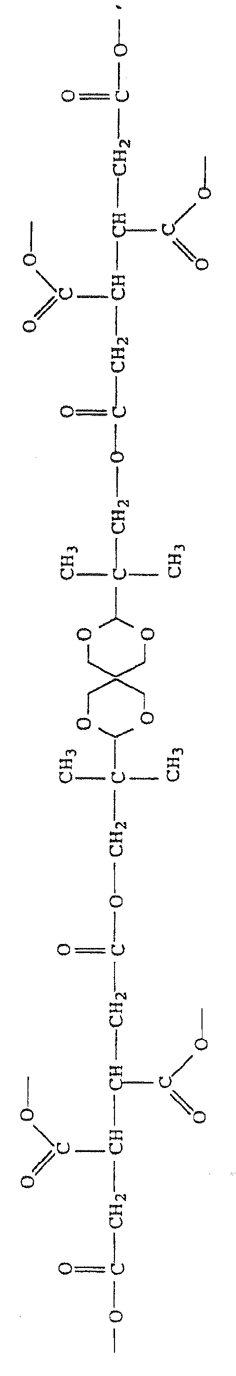 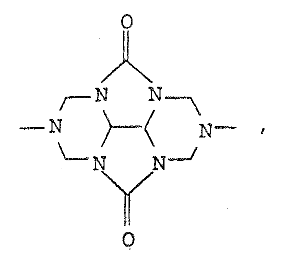 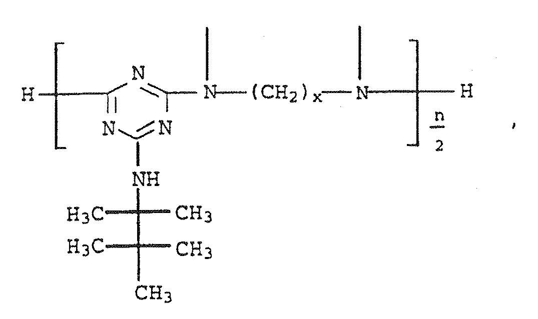 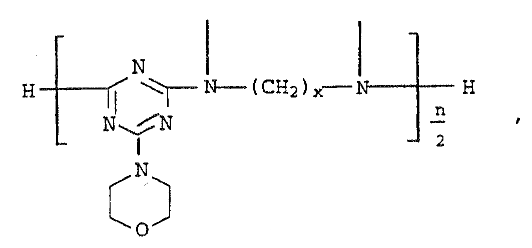 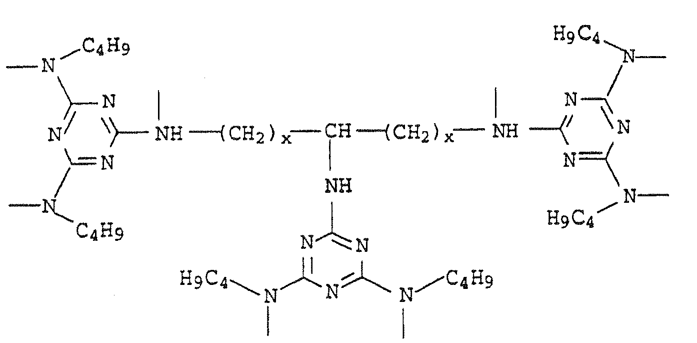
ou 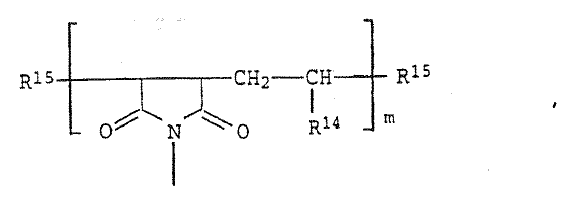 où
R¹¹ représente un groupe alkyle en C₁ à C₁₂, ou -(CH₂)_{z}- COOR¹⁰,
R¹² représente un atome d'hydrogène ou un groupe alkyle en C₁ à C₁₈,
R¹³ représente un groupe alkyle en C₁ à C₁₈, vinyle ou isopropényle,
R¹⁴ représente un groupe alkyle en C₈ à C₂₂,
R¹⁵ représente un atome d'hydrogène ou un résidu organique, comme celui qui se forme habituellement lors de la polymérisation par voie radicalaire des monomères de départ (A),
k vaut 0 ou 1,
x vaut de 1 à 12 et
n représente un nombre pair m.

7. Mélange de substances (B) selon les revendications 1, 2, 5 à 6, lequel contient en plus des composants (i) et (ii), encore un ou plusieurs co-stabilisants choisis dans le groupe formée par les composés nitroso aromatiques, les phénothiazines, les quinones, les hydroquinones et leurs éthers, les phénols et leurs éthers, les hydroxylamines et les phénylène-diamines.

8. Procédé pour empêcher la polymérisation précoce de monomères contenant des groupes vinyle pendant la purification ou la distillation, **caractérisé en ce que** l'on ajoute aux monomères, un mélange (B) selon les revendications 1, 2, 5 à 7, en une quantité efficace, avant ou pendant la purification ou la distillation.

9. Procédé selon la revendication 8, **caractérisé en ce que** l'on ajoute aux monomères, les composants du mélange (B) selon les revendications 1, 2, 5 à 7, individuellement, à chaque fois en une quantité efficace, avant la purification ou la distillation.

10. Procédé selon la revendication 9, **caractérisé en ce que** l'on ajoute les composants en des positions spatialement différentes.

11. Utilisation du mélange (B) selon les revendications 1, 2, 5 à 7 pour empêcher la polymérisation précoce de monomères polymérisables par voie radicalaire.
